(19) Europäisches Patentamt / European Patent Office / Office européen des brevets

(11) **EP 3 201 316 B1**

(12) **EUROPEAN PATENT SPECIFICATION**

(45) Date of publication and mention
of the grant of the patent:
**19.02.2020   Bulletin 2020/08**

(21) Application number: **15775162.9**

(22) Date of filing: **30.09.2015**

(51) Int Cl.:
*C12N 1/20* (2006.01)       *C12N 9/88* (2006.01)

(86) International application number:
**PCT/EP2015/072594**

(87) International publication number:
**WO 2016/050861 (07.04.2016 Gazette 2016/14)**

(54) **METHOD OF CULTIVATING MICROORGANISMS HAVING NITRILE HYDRATASE ACTIVITY**

VERFAHREN ZUR KULTIVIERUNG VON MIKROORGANISMEN MIT NITRILHYDRATASEAKTIVITÄT

PROCÉDÉ DE CULTURE DE MICRO-ORGANISME PRÉSENTANT UNE ACTIVITÉ DE NITRILE HYDRATASE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **30.09.2014  EP 14187144**

(43) Date of publication of application:
**09.08.2017   Bulletin 2017/32**

(73) Proprietor: **Solenis Technologies Cayman, L.P.
KY1-1104, Cayman (KY)**

(72) Inventors:
• **HAGE, Kerstin
67346 Speyer (DE)**
• **SCHWALB, Carsten
67310 Hettenleidelheim (DE)**
• **SPRÖTE, Petra
68163 Mannheim (DE)**
• **ÖDMAN, Peter
67434 Neustadt (DE)**
• **BALDENIUS, Kai-Uwe
68159 Mannheim (DE)**
• **ERNST, Burkhard
31180 Giesen / Hasede (DE)**
• **STEIGELMANN, Gunter
76835 Rhodt (DE)**

• **FREYER, Stephan
67434 Neustadt (DE)**
• **BOLLSCHWEILER, Claus
69118 Heidelberg (DE)**
• **BRAUN, Michael Günter
69123 Heidelberg (DE)**
• **DAEUWEL, Juergen
69124 Heidelberg (DE)**

(74) Representative: **LKGlobal UK Ltd.
Cambridge House, Henry Street
Bath BA1 1BT (GB)**

(56) References cited:
**EP-A2- 0 137 076       WO-A1-2004/085628
WO-A1-2008/127997     WO-A1-2011/099165
WO-A1-2014/102703     WO-A2-2009/009117
US-A1- 2014 220 644**

• **LAWRENCE J. MERSINGER ET AL:
"Production of Acrylamide using
Alginate-ImmobilizedE. coli
ExpressingComamonas testosteroni
5-MGAM-4D Nitrile Hydratase", ADVANCED
SYNTHESIS & CATALYSIS, vol. 347, no. 7-8, 1
June 2005 (2005-06-01), pages 1125-1131,
XP055241753, DE ISSN: 1615-4150, DOI:
10.1002/adsc.200505039**

**Description**

The present invention relates to methods for cultivating a nitrile hydratase producing microorganism, compositions for cultivating a nitrile hydratase producing microorganism, and use of compositions comprising a saccharide and an organic acid for cultivating a nitrile hydratase producing microorganism. The composition provided in and to be employed in context with the present invention is particularly suitable for inducing both, growth and nitrile hydratase production of corresponding microorganisms.

Polyacrylamide is widely used as flocculants, as thickener in the paper industry, as additive in tertiary oil recovery, and many other fields. The raw material for polyacrylamide is typically its monomer acrylamide. In principal, there exist two different methods to produce acrylamide in industrial scales: Chemical synthesis and biological synthesis, wherein the biological synthesis methods are more and more on the rise due to milder reaction conditions and inherent process safety. Due to the milder reaction conditions the absence of copper catalyst and the quantitative conversion of the nitrile, expensive downstream processing steps such as distillation or ion exchange can be avoided in the biological synthesis, thus resulting in cheaper plants with drastically reduced plant footprint.

Both synthesis methods use acrylonitrile as starting substance. While the chemical synthesis method uses copper catalysts (e.g., US4048226, US3597481), the biological synthesis method employs biocatalysts to hydrate acrylonitrile in order to obtain acrylamide. Generally, such biocatalysts are microorganisms which are able to produce the enzyme nitrile hydratase (IUBMB nomenclature as of September 30, 2014: EC 4.2.1.84; CAS-No. 2391-37-5; also referred to as, e.g., NHase). Nitrile hydratase producing microorganisms are largely distributed in the environment and comprise, *inter alia,* representatives of *Rhodococcus rhodochrous, Rhodococcus pyridinovorans, Rhodococcus erythropolis, Rhodococcus equi, Rhodococcus ruber, Rhodococcus opacus, Aspergillus niger, Acidovorax avenae, Acidovorax facilis, Agrobacterium tumefaciens, Agrobacterium radiobacter, Bacillus subtilis, Bacillus pallidus, Bacillus smithii, Bacillus sp BR449, Bradyrhizobium oligotrophicum, Bradyrhizobium diazoefficiens, Bradyrhizobium japonicum, Burkholderia cenocepacia, Burkholderia gladioli, Klebsiella oxytoca, Klebsiella pneumonia, Klebsiella variicola, Mesorhizobium ciceri, Mesorhizobium opportunistum, Mesorhizobium sp F28, Moraxella, Pantoea endophytica, Pantoea agglomerans, Pseudomonas chiororaphis, Pseudomonas putida, Rhizobium, Rhodopseudomonas palustris, Serratia liquefaciens, Serratia marcescens, Amycolatopsis, Arthrobacter, Brevibacterium sp CH1, Brevibacterium sp CH2, Brevibacterium sp R312, Brevibacterium imperiale, Corynebacterium nitrilophilus, Corynebacterium pseudodiphteriticum, Corynebacterium glutamicum, Corynebacterium hoffmanii, Microbacterium imperiale, Microbacterium smegmatis, Micrococcus luteus, Nocardia globerula, Nocardia rhodochrous, Pseudonocardia thermophila, Trichoderma, Myrothecium verrucaria, Aureobasidium pullulans, Candida famata, Candida guilliermondii, Candida tropicalis, Cryptococcus fiavus, Cryptococcus sp UFMG- Y28, Debaryomyces hanseii, Geotrichum candidum, Geotrichum sp JR1, Hanseniaspora, Kluyveromyces thermotolerans, Pichia kluyveri, Rhodotorula glutinis, Comomonas testosteroni, Pyrococcus abyssi, Pyrococcus furiosus, and Pyrococcus horikoshii.* (see, e.g., Prasad, Biotechnology Advances (2010), 28(6): 725-741; FR2835531). The enzyme nitrile hydratase is either iron or cobalt-dependent *(i.e.* it possesses either an iron or a cobalt atom coordinated in its activity center) which is particularly characterized by its ability to catalyze conversion of acrylonitrile to obtain acrylamide by hydrating acrylonitrile (Kobayashi, Nature Biotechnology (1998),16: 733 - 736).

The capability of microorganisms to act as biocatalysts for converting acrylonitrile to acrylamide is basically dependent on two parameters: Sufficient growth of the microorganisms and their production rate of nitrile hydratase. Known fermentation methods for cultivation of nitrile hydratase producing microorganisms face several different difficulties, e.g. high concentrations of carbon or nitrogen sources suitable for growth may inhibit the enzyme production, so called "catabolite repression" (Leonova, Applied Biochem Biotechnol (2000), 88: 231-241) or inhibiting effects of metal ion cofactors (EP-B1 1283256; Pei, Biotechnology letters (2013), 35: 1419-1424). These difficulties result in low nitrile hydratase activity rates within a given period of time, either due to low enzyme production rates (*i.e.* the microorganisms are primarily growing but still exhibit low enzyme production rates), or due to low microorganism growth rates (*i.e.* the microorganisms exhibit high production rates, but there are still too few microorganisms).

Thus, there is a need for a method of cultivating nitrile hydratase producing microorganisms which allows obtaining high yields of nitrile hydratase activity in short time.

This objective technical problem has been overcome by the present invention as defined in the claims and as described and exemplified herein below.

The gist of the present invention lies in the surprising finding that combinations of saccharides and organic acids increase growth and production rates of nitrile hydratase according to nitrile hydratase activity levels. Without being bound by theory, it is believed that the import of saccharide into the cells is improved by the organic acids.

Thus, the present invention relates to a method for cultivating a nitrile hydratase (NHase) producing microorganism, comprising the steps of:

(a) contacting said microorganism with an aqueous composition comprising a saccharide (i) and an organic acid (ii);
(b) cultivating said microorganism in said aqueous composition of (a).

The present invention also relates to microorganisms obtained from or obtainable by the cultivation method described and provided herein.

The present invention also relates to a composition for cultivating a nitrile hydratase producing microorganism, said composition comprising a saccharide (i) and an organic acid (ii).

The present invention further relates to the use of a composition comprising a saccharide (i) and an organic acid (ii) for cultivating a nitrile hydratase producing microorganism.

In the following, when further defining and specifying single features (such as, e.g., nitrile hydratase, microorganism, composition, saccharide, organic acid, etc.) of the method, the composition and the use of such composition as described and provided according to the present invention, such definitions and specifications shall apply to all, the inventive method, the inventive composition and the inventive use of such composition as described and provided herein.

As has turned out in line with the findings of the present invention, some saccharides appear to be more potent in combination with organic acids to increase nitrile hydratase activity (e.g., allowing high growth of microorganisms and high enzyme production) than others. Accordingly, in one embodiment, the saccharide (i) comprised by the composition as provided and to be employed in context with the present invention is a monosaccharide. Furthermore, in context with the present invention, the saccharide (i) may be a saccharide wherein the number of carbon atoms is at least 5, preferably at least 6. In one embodiment, the number of carbon atoms in the saccharide (i) is 6. Specific examples for the saccharide contained in the composition provided herein and to be employed in context with the present invention include glucose, fructose, ribose, and mannose; preferably glucose, fructose or mannose. In one embodiment, the saccharide (i) is glucose.

The organic acid (ii) contained in the composition provided herein and to be employed in context with the present invention may be, for example, an organic acid which comprises not more than 3 carboxyl groups, preferably not more than 2 carboxyl groups, and most preferably not more than 1 carboxyl group. Such organic acids have been found in line with the present invention as particularly potent for increasing the nitrile hydratase activity as further described and exemplified herein and in the examples. The organic acid (ii) is not generally limited in size, but may comprise, e.g., not more than 6 carbon atoms, preferably not more than 4 carbon atoms, and most preferably not more than 3 carbon atoms. Particular examples for organic acids in context with the present invention include lactic acid, tartaric acid, citric acid, malic acid, and succinic acid. In one embodiment of the present invention, the organic acid (ii) is selected from the group consisting of lactic acid, tartaric acid, citric acid, malic acid, and succinic acid; preferably from the group consisting of lactic acid, tartaric acid, malic acid, and succinic acid; more preferably from the group consisting of lactic acid, tartaric acid, and malic acid; and most preferably it is lactic acid.

In one specific embodiment of the present invention, the saccharide (i) contained in the composition provided herein and to be employed in context with the present invention is glucose, and the organic acid (ii) is lactic acid.

In context with the method of cultivating a nitrile producing microorganism as provided and described herein, it is possible to first bring the microorganism into contact with a composition comprising a saccharide (i) (and not yet an organic acid (ii)) according to step (a), then start cultivating the microorganism according to step (b), and only then add an organic acid (ii) to the composition during cultivation of the microorganism. Also, *vice versa,* in context with the method of cultivating a nitrile producing microorganism as provided and described herein, it is possible to first bring the microorganism into contact with a composition comprising an organic acid(i) (and not yet a saccharide (i)) according to step (a), then start cultivating the microorganism according to step (b), and only then add a saccharide (i) to the composition during cultivation of the microorganism. In other words, it is not necessary that both components saccharide (i) and organic acid (ii) are present in the composition contacted with the microorganism right from the beginning of cultivation. Rather, it is also possible that one or both components (i) and (ii) can be added to the composition after the cultivation of the nitrile hydratase producing microorganism has begun, as long as in the end the microorganism is cultivated in a composition comprising both components in order to achieve the desired effect of increasing nitrile hydratase activity.

The ratio of saccharide (i) and organic acid (ii) as contained in the composition provided herein and to be employed in context with the present specification is not generally limited. Possible examples of ratios (always indicated herein as weight/weight-ratio) between saccharide (i) and organic acid (ii) as contained in the composition provided herein and to be employed in context with the present invention are 1:9 to 9:1; 2:8 to 8:2; 2,5:7,5 to 7;5:2,5; 3:7 to 7:3; 3,5:6,5 to 6,5:3,5; 4:6 to 6:4; 4,5;5,5 to 5,5:4;5; or 1:1. In the specification the ratio between saccharide (i) and organic acid (ii) in the composition is between 2:8 and 9:1, preferably between 3:7 and 9:1, more preferably 3:7 and 8:2, more preferably 3:7 and 7,5:2,5, and most preferably between 3:7 and 7:3 or between 1:1 and 7:3. In context with the present invention, these ratios refer to the respective weights of saccharide (i) and organic acid (ii) as added to the composition provided herein and to be employed in context with the present invention for cultivating the nitrile hydratase producing microorganism. For example, the ratios in the composition may vary during cultivation of the nitrile hydratase producing microorganism as both components may be independently consumed or converted quicker or slower, or as one or both of the components are added in additional amounts throughout the course of the cultivation.

In one specific embodiment of the present invention, the saccharide (i) contained in the composition provided herein and to be employed in context with the present invention is glucose, the organic acid (ii) is lactic acid, and the ration between saccharide (i) and organic acid (ii) in the composition is between 3:7 and 7:3.

The composition comprising a saccharide (i) and an organic acid (ii) as described and provided and to be employed according to the present invention may further comprise additional components which are useful for compositions for cultivating microorganisms as known in the art. Most preferably, in context with the present invention, said composition is an aqueous composition. Further components may include, e.g., a nitrogen source (e.g., an ammonium or nitrate salt, an organic acid such as glutamic acid, a yeast extract, or a protein hydrolysate), a phosphorous source (e.g., a phosphate salt, which may also provide buffering capacity), a sulfur source (e.g., a sulfate salt), a source of cobalt and/or iron (as co-factor for the NHase), inductors for expression of nitrile hydratase such as urea, sources of other nutrients such as magnesium, calcium, zinc, manganese, copper, as well as vitamins.

The cultivation of nitrile hydratase producing microorganisms as described herein, particularly in context with step (b) of the method provided herein, can be conducted in a usual manner known to the person skilled in the art. That is, the composition provided herein and to be employed in this context should contain - beside a saccharide (i) and an organic acid (ii) as described herein - additional components necessary to allow growth and maintenance of the microorganism to be cultivated. Furthermore, other parameters such as temperature, pH, dissolved oxygen concentration, pressure, aeration and agitation, should be set to allow growth and maintenance of the microorganisms to be cultivated in context with the present invention. In this context, typical temperatures for cultivating microorganisms as described and exemplified herein may lie between 30 °C and 40 °C, preferably between 35 °C and 38 °C, most preferably between 36.5 °C and 37.5 °C, particularly at 37 °C. However, also other temperatures may be set depending on the microorganism to be cultivated in context with the present invention. The pH should be kept between 6 and 8, preferably between 6,5 and 7,5, either by providing a buffer in the fermentation medium or by feed-back controlled addition of a strong acid and/or base. Typical dissolved oxygen concentration values may lie between 5% to 75%, preferably 20% to 40% of the saturation concentration.

In context with the present invention, after cultivation of the nitrile hydratase producing microorganism according to the cultivation method described and provided herein, the microorganism may be collected from the cultivation composition and dried or otherwise accumulated. Optionally, the cell suspension may be concentrated before drying, e.g. via centrifugation or crossflow filtration (in microfiltration or ultrafiltration mode). The cells may also be washed with water or a buffer solution before drying in order to remove residual substances from the fermentation broth. For example, the microorganism may then be dried via spray-drying, fluidized-bed drying, spray granulation or freeze drying after cultivation, preferably by spray- or freeze drying. For example, the cells can be spray-dried under mild conditions, such as with an inlet temperature of 80 to 150 °C, preferably 90 to 120 °C, and an outlet temperature of, e.g., 35 to 65 °C, preferably 40 to 50 °C, to a residual water content of 1 to 10 %, preferably 4 to 8% by weight.

In context with the present invention, the nitrile hydratase producing microorganism to be cultivated with the composition comprising a saccharide (i) and an organic acid (ii) may be any microorganism which is able to produce the enzyme nitrile hydratase as described herein. The enzyme nitrile hydratase is either iron- or cobalt-dependent (i.e. it possesses either an iron or a cobalt atom coordinated in its activity center) which is particularly characterized by its ability to catalyze conversion of acrylonitrile to obtain acrylamide by hydrating acrylonitrile (Kobayashi, Nature Biotechnology (1998),16: 733 - 736). Such microorganisms to be employed in context with the present invention may be microorganisms which are naturally able to produce nitrile hydratase, *i.e.* which naturally contain a gene encoding nitrile hydratase. Such microorganisms may also be microorganisms which are naturally able to produce nitrile hydratase, and which are further genetically engineered, e.g., to increase production of nitrile hydratase, or to increase stability and/or export of nitrile hydratase. Such microorganisms may also be microorganisms which are not naturally able to produce nitrile hydratase, and which are further genetically engineered to stably express and produce nitrile hydratase. In this context, microorganisms naturally able to produce nitrile hydratase are known in the art and comprise, *inter alia,* representatives of *Rhodococcus rhodochrous, Rhodococcus pyridinovorans, Rhodococcus erythropolis, Rhodococcus equi, Rhodococcus ruber, Rhodococcus opacus, Aspergillus niger, Acidovorax avenae, Acidovorax facilis, Agrobacterium tumefaciens, Agrobacterium radiobacter, Bacillus subtilis, Bacillus pallidus, Bacillus smithii, Bacillus sp BR449, Bradyrhizobium oligotrophicum, Bradyrhizobium diazoefficiens, Bradyrhizobium japonicum, Burkholderia cenocepacia, Burkholderia gladioli, Klebsiella oxytoca, Klebsiella pneumonia, Klebsiella variicola, Mesorhizobium ciceri, Mesorhizobium opportunistum, Mesorhizobium sp F28, Moraxella, Pantoea endophytica, Pantoea agglomerans, Pseudomonas chlororaphis, Pseudomonas putida, Rhizobium, Rhodopseudomonas palustris, Serratia liquefaciens, Serratia marcescens, Amycolatopsis, Arthrobacter, Brevibacterium sp CH1, Geobacillus sp RAPc8, Brevibacterium sp CH2, Brevibacterium sp R312, Brevibacterium imperiale, Corynebacterium nitrilophilus, Corynebacterium pseudodiphteriticum, Corynebacterium glutamicum, Corynebacterium hoffmanii, Microbacterium imperiale, Microbacterium smegmatis, Micrococcus luteus, Nocardia globerula, Nocardia rhodochrous, Pseudonocardia thermophila, Trichoderma, Myrothecium verrucaria, Aureobasidium pullulans, Candida famata, Candida guilliermondii, Candida tropicalis, Cryptococcus flavus, Cryptococcus sp UFMG- Y28, Debaryomyces hanseii, Geotrichum candidum, Geotrichum sp JR1, Hanseniaspora, Kluyveromyces thermotolerans, Pichia kluyveri, Rhodotorula glutinis, Comomonas testosteroni, Pyrococcus abyssi, Pyrococcus furiosus,* and *Pyrococcus horikoshii..* Also, microorganisms not naturally expressing nitrile hydratase such as *Escherichia (e.g., Escherichia coli)* may be employed once they have been genetically engineere such as to stably express and produce nitrile hydrates. In one embodiment of the present invention, the nitrile hydratase producing microorganism is a representative of the genus *Rhodococcus,* e.g., of the species *Rhodococcus rhodochrous* or *Rhodococcus pyridinovorans.* In this context, examples for representatives of the species *Rhodococcus rhodochrous* may comprise the strains as deposited under no. NCIMB 41164, FERM-BP 1478 (J1), M33 or M8. Furthermore, in context with the present invention, nitrile hydratase producing microorganisms may be genetically engineered microorganisms which naturally do not contain a gene encoding a nitrile hydratase but which have been manipulated such as to contain a polynucleotide encoding a nitrile hydratase (e.g., via transformation, transduction, transfection, conjugation, or other methods suitable to transfer or insert a polynucleotide into a cell as known in the art; cf. Sambrook and Russell 2001, Molecular Cloning: A Laboratory Manual, CSH Press, Cold Spring Harbor, NY, USA), thus enabling the microorganisms to produce the nitrile hydratase enzyme. For this purpose, it may further be required to insert additional polynucleotides which may be necessary to allow transcription and translation of the nitrile hydratase gene or mRNA, respectively. Such additional polynucleotides may comprise, *inter alia,* promoter sequences, polyT- or polyU-tails, or replication origins or other plasmid-control sequences. In this context, such genetically engineered microorganisms which naturally do not contain a gene encoding a nitrile hydratase but which have been manipulated such as to contain a polynucleotides encoding a nitrile hydratase may be prokaryotic or eukaryotic microorganisms. Examples for such prokaryotic microorganisms include, e.g., representatives of the species *Escherichia coli.* Examples for such eukaryotic microorganisms include, e.g., yeast (e.g., *Saccharomyces cerevisiae).*

In one specific embodiment of the present invention, the saccharide (i) contained in the composition provided herein and to be employed in context with the present invention is glucose, the organic acid (ii) is lactic acid, and the nitrile hydratase producing microorganism is a *Rhodococcus rhodochrous,* e.g. NCIMB 41164 or FERM-BP 1478.

In another specific embodiment of the present invention, the nitrile hydratase producing microorganism is a *Rhodococcus rhodochrous,* and the ration between saccharide (i) and organic acid (ii) in the composition is between 3:7 and 7:3.

In a further specific embodiment of the present invention, the saccharide (i) contained in the composition provided herein and to be employed in context with the present invention is glucose, the organic acid (ii) is lactic acid, the nitrile hydratase producing microorganism is a *Rhodococcus rhodochrous,* and the ration between saccharide (i) and organic acid (ii) in the composition is between 3:7 and 7:3.

In context with the present invention, the term "nitrile hydratase" means the enzyme nitrile hydratase (also referred to herein as "NHase") which is capable of catalyzing hydration of acrylonitrile to acrylamide, *i.e.* which has nitrile hydratase activity. In context with the present invention, activity of an nitrile hydratase in the sense of the present invention can be determined as follows: First reacting 100 µl of a cell suspension, cell lysate, dissolved enzyme powder or any other preparation containing the supposed nitrile hydratase with 875 µl of an 50 mM potassium phosphate buffer and 25 µl of acrylonitrile at 25 °C on an eppendorf tube shaker at 1,000 rpm for 10 minutes. After 10 minutes of reaction time, samples are drawn and immediately quenched by adding the same volume of 1.4% hydrochloric acid. After mixing of the sample, cells are removed by centrifugation for 1 minute at 10,000 rpm and the amount of acrylamide formed is determined by analyzing the clear supernatant by HPLC. The concentration of acrylamide shall be between 0.25 and 1.25 mmol/l - if necessary, the sample has to be diluted accordingly and the conversion has to be repeated. The enzyme activity is deduced from the concentration of acrylamide by dividing the acrylamide concentration derived from HPLC analysis by the reaction time, which has been 10 minutes and by multiplying this value with the dilution factor between HPLC sample and original sample. Activities >10 U/ml, preferably >100 U/ml, more preferably >1,000 U/ml indicate the presence of a functionally expressed nitrile hydratase and are considered as nitrile hydratase activity and, thus, as a nitrile hydrates enzyme in context with the present invention. For example, nitrile hydratase as used herein also comprises enzymes classified under IUBMB nomenclature (as of September 30, 2014) as EC 4.2.1.84 or as CAS-No. 2391-37-5, as well as modified or enhanced enzymes which are, e.g., capable of converting a nitrile compound (e.g. acrylonitrile) to an amide compound (e.g. acrylamide) more quickly, or which can be produced at a higher yield/time-ratio, or which are more stable, as long as they are capable to catalyze conversion (*i.e.* hydration) of a nitrile compound (e.g. acrylonitrile) to an amide compound (e.g. acrylamide). In context with the present invention, the nitrile hydratase may be a polypeptide encoded by a polynucleotide which comprises or consists of a nucleotide sequence which is at least 70%, preferably at least 75%, more preferably at least 80%, more preferably at least 85%, more preferably at least 90%, more preferably at least 95%, more preferably at least 96%, more preferably at least 97%, more preferably at least 98%, more preferably at least 99%, more preferably at least 99,5%, and most preferably 100% identical to the nucleotide sequence of SEQ ID NO: 1 (alpha-subunit of nitrile hydratase of *R. rhodochrous:*
GTGAGCGAGCACGTCAATAAGTACACGGAGTACGAGGCACGTACCAAGGCGATCGAAACCTTGCTGTACGAGC
GAGGGCTCATCACGCCCGCCGCGGTCGACCGAGTCGTTTCGTACTACGAGAACGAGATCGGCCCGATGGGCG
GTGCCAAGGTCGTGGCCAAGTCCTGGGTGGACCCTGAGTACCGCAAGTGGCTCGAAGAGGACGCGACGGCCG
CGATGGCGTCATTGGGCTATGCCGGTGAGCAGGCACACCAAATTTCGGCGGTCTTCAACGACTCCCAAACGCAT
CACGTGGTGGTGTGCACTCTGTGTTCGTGCTATCCGTGGCCGGTGCTTGGTCTCCCGCCCGCCTGGTACAAGA
GCATGGAGTACCGGTCCCGAGTGGTAGCGGACCCTCGTGGAGTGCTCAAGCGCGATTTCGGTTTCGACATCCC

CGATGAGGTGGAGGTCAGGGTTTGGGACAGCAGCTCCGAAATCCGCTACATCGTCATCCCGGAACGGCCGGCC
GGCACCGACGGTTGGTCCGAGGAGGAGCTGACGAAGCTGGTGAGCCGGGACTCGATGATCGGTGTCAGTAAT
GCGCTCACACCGCAGGAAGTGATCGTATGA) and/or to the nucleotide sequence of SEQ ID NO: 3 (beta-subunit of nitrile hydratase of *R. rhodochrous:*

ATGGATGGTATCCACGACACAGGCGGCATGACCGGATACGGACCGGTCCCCTATCAGAAGGACGAGCCCTTCT
TCCACTACGAGTGGGAGGGTCGGACCCTGTCAATTCTGACTTGGATGCATCTCAAGGGCATATCGTGGTGGGAC
AAGTCGCGGTTCTTCCGGGAGTCGATGGGGAACGAAACTACGTCAACGAGATTCGCAACTCGTACTACACCCA
CTGGCTGAGTGCGGCAGAACGTATCCTCGTCGCCGACAAGATCATCACCGAAGAAGAGCGAAAGCACCGTGTG
CAAGAGATCCTTGAGGGTCGGTACACGGACAGGAAGCCGTCGCGGAAGTTCGATCCGGCCCAGATCGAGAAGG
CGATCGAACGGCTTCACGAGCCCCACTCCCTAGCGCTTCCAGGAGCGGAGCCGAGTTTCTCTCTCGGTGACAA
GATCAAAGTGAAGAGTATGAACCCGCTGGGACACACACGGTGCCCGAAATATGTGCGGAACAAGATCGGGGAA
ATCGTCGCCTACCACGGCTGCCAGATCTATCCCGAGAGCAGCTCCGCCGGCCTCGGCGACGATCCTCGCCCGC
TCTACACGGTCGCGTTTTCCGCCCAGGAACTGTGGGGCGACGACGGAAACGGGAAGACGTAGTGTGCGTCGA
TCTCTGGGAACCGTACCTGATCTCTGCGTGA), provided that the polypeptide encoded by said polynucleotide is capable of catalyzing hydration of acrylonitrile to acrylamide (*i.e.* has nitrile hydratase activity) as described and exemplified herein. Also in the context with the present invention, the nitrile hydratase may be a polypeptide which comprises or consists of an amino acid sequence which is at least 70%, preferably at least 75%, more preferably at least 80%, more preferably at least 85%, more preferably at least 90%, more preferably at least 95%, more preferably at least 96%, more preferably at least 97%, more preferably at least 98%, more preferably at least 99%, more preferably at least 99,5%, and most preferably 100% identical to the amino acid sequence of SEQ ID NO: 2 (alpha-subunit of nitrile hydratase of *R. rhodochrous:* VSEHVNKYTE YEARTKAIET LLYERGLITP AAVDRVVSYY ENEIGPMGGA KVVAKSWVDP EYRKWLEEDA TAAMASLGYA GEQAHQISAV FNDSQTHHVV VCTLCSCYPW PVLGLPPAWY KSMEYRSRVV ADPRGVLKRD FGFDIPDEVE VRVWDSSSEI RYIVIPERPA GTDGWSEEEL TKLVSRDSMI GVSNALTPQE VIV) and/or to the amino acid sequence of SEQ ID NO: 4 (beta-subunit of nitrile hydratase of *R. rhodochrous:* MDGIHDTGGM TGYGPVPYQK DEPFFHYEWE GRTLSILTWM HLKGISWWDK SRFFRESMGN ENYVNEIRNSY YTHWLSAAE RILVADKIIT EEERKHRVQE ILEGRYTDRK PSRKFDPAQI EKAIERLHEP HSLALPGAEP SFSLGDKIKV KSMNPLGHTR CPKYVRNKIG EIVAYHGCQI YPESSSAGLG DDPRPLYTVA FSAQELWGDD GNGKDVVCVD LWEPYLISA), provided that said polypeptide is capable of catalyzing hydration of acrylonitrile to acrylamide as described and exemplified herein.

The level of identity between two or more sequences (e.g., nucleic acid sequences or amino acid sequences) can be easily determined by methods known in the art, e.g., by BLAST analysis. Generally, in context with the present invention, if two sequences (e.g., polynucleotide sequences or amino acid sequences) to be compared by, e.g., sequence comparisons differ in identity, then the term "identity" may refer to the shorter sequence and that part of the longer sequence that matches said shorter sequence. Therefore, when the sequences which are compared do not have the same length, the degree of identity may preferably either refer to the percentage of nucleotide residues in the shorter sequence which are identical to nucleotide residues in the longer sequence or to the percentage of nucleotides in the longer sequence which are identical to nucleotide sequence in the shorter sequence. In this context, the skilled person is readily in the position to determine that part of a longer sequence that matches the shorter sequence. Furthermore, as used herein, identity levels of nucleic acid sequences or amino acid sequences may refer to the entire length of the respective sequence and is preferably assessed pair-wise, wherein each gap is to be counted as one mismatch. These definitions for sequence comparisons (e.g., establishment of "identity" values) are to be applied for all sequences described and disclosed herein.

Moreover, the term "identity" as used herein means that there is a functional and/or structural equivalence between the corresponding sequences. Nucleic acid/amino acid sequences having the given identity levels to the herein-described particular nucleic acid/amino acid sequences may represent derivatives/variants of these sequences which, preferably, have the same biological function. They may be either naturally occurring variations, for instance sequences from other varieties, species, etc., or mutations, and said mutations may have formed naturally or may have been produced by deliberate mutagenesis. Furthermore, the variations may be synthetically produced sequences. The variants may be naturally occurring variants or synthetically produced variants or variants produced by recombinant DNA techniques. Deviations from the above-described nucleic acid sequences may have been produced, e.g., by deletion, substitution, addition, insertion and/or recombination. The term "addition" refers to adding at least one nucleic acid residue/amino acid to the end of the given sequence, whereas "insertion" refers to inserting at least one nucleic acid residue/amino acid within a given sequence. The term "deletion" refers to deleting or removal of at least one nucleic acid residue or amino acid residue in a given sequence. The term "substitution" refers to the replacement of at least one nucleic acid residue/amino acid residue in a given sequence. Again, these definitions as used here apply, *mutatis mutandis,* for all sequences provided and described herein.

Generally, as used herein, the terms "polynucleotide" and "nucleic acid" or "nucleic acid molecule" are to be construed synonymously. Generally, nucleic acid molecules may comprise *inter alia* DNA molecules, RNA molecules, oligonucleotide thiophosphates, substituted ribo-oligonucleotides or PNA molecules. Furthermore, the term "nucleic acid molecule" may refer to DNA or RNA or hybrids thereof or any modification thereof that is known in the art (see, e.g., US 5525711, US 471 1955, US 5792608 or EP 302175 for examples of modifications). The polynucleotide sequence may be single- or double- stranded, linear or circular, natural or synthetic, and without any size limitation. For instance, the polynucleotide sequence may be genomic DNA, cDNA, mitochondrial DNA, mRNA, antisense RNA, ribozymal RNA or a DNA encoding such RNAs or chimeroplasts (Gamper, Nucleic Acids Research, 2000, 28, 4332 - 4339). Said polynucleotide sequence may be in the form of a vector, plasmid or of viral DNA or RNA. Also described herein are nucleic acid molecules which are complementary to the nucleic acid molecules described above and nucleic acid molecules which are able to hybridize to nucleic acid molecules described herein. A nucleic acid molecule described herein may also be a fragment of the nucleic acid molecules in context of the present invention. Particularly, such a fragment is a functional fragment. Examples for such functional fragments are nucleic acid molecules which can serve as primers.

Generally, the present invention relates to all the embodiments described herein as well as to all permutations and combinations thereof. Any particular aspects or embodiments described herein must not be construed as limiting the scope of the present invention on such aspects or embodiments.

The following Examples illustrate the present invention. Yet, the present invention must not be construed as being limited by the following Examples.

**Examples**

**Example 1**

**Cultivation of *Rhodococcus* ssp.**

Different sugar / organic acid ratios were evaluated for different strains of *Rhodococcus* (*R. rhodochrous* "CIBA" (NCIMB 41164) and *R. rhodochrous* "J1" (FERM BP-1478)), in a microfermentation system.

The basic cultivation medium contained yeast extract (Biospringer) (1.25 g/l), $KH_2PO_4$ (10 g/l), $K_2HPO_4$ (10 g/l), $(NH_4)_2SO_4$ (1 g/l), $MgSO_4*7H_2O$ (0.375 g/l), $CaCl_2*2H_2O$ (25 mg/l), Trace element solution (2.5 g/l) (see below), $Co(NO_3)_2$ (31.2 mg/l), anti-foam P2000 (BASF) (62.5 mg/l), urea (7 g/l), and ammonium glutamate (2.5 g/l) in $H_2O$. Trace element solution: Citric acid mono hydrate (40g/l), $ZnSO_4*7$ $H_2O$ (11 g/l), $(NH_4)_2Fe(SO_4)_2*6H_2O$ (8.5 g/l), $MnSO_4*H_2O$ (3 g/l), and $CuSO_4*5$ $H_2O$ (0.8 g/l) in $H_2O$.

The medium was then supplemented with 10 g/l of carbon source (saccharide, organic acid or mixture of both as indicated in the Tables below). pH was adjusted to 6.6 using $H_3PO_4$ or NaOH. The medium was inoculated directly from cryo stocks to OD 0.05 (measured at 600 nm). Cultivation was carried out at 37 °C for 64 h at 1,100 rpm in a BioLector micro fermentation system (M2P Labs, Baesweiler, Germany) using 48-well flower plates with a working volume of 1.5 ml. After cultivation, samples were drawn, quenched with hydrochloric acid and nitrile hydratase activity was determined as described in Example 2.

In the tables below, relative nitrile hydratase activities for different strains and saccharide/acid ratios are provided. Nitrile hydratase activities achieved with the saccharide as sole carbon source were set to 100%.

**Table 1:** *R. rhodochrous* "CIBA"

| Saccharide | Acid | Saccharide / Acid ratio by weight | | | | | | |
| | | Only acid | 1 / 9 | 3 / 7 | 1 / 1 | 7 / 3 | 1 / 9 | Only saccharide |
|---|---|---|---|---|---|---|---|---|
| Glucose | Lactic | N/A | 24% | 1037% | 1242% | 748° | 422% | 100% |
| | Tartaric | 173% | 244% | 1354% | 1044% | 737° | 191% | 100% |
| | Malic | 197% | 392% | 1199% | 2596% | 1709% | 801% | 100% |
| | Citric | 164% | 190% | 161% | 1433% | 635% | 57% | 100% |
| | Succinic | 150% | 510% | 1365% | 2303% | 122E | 547% | 100% |

(continued)

| Saccharide | Acid | Saccharide / Acid ratio by weight | | | | | | |
| | | Only acid | 1 / 9 | 3 / 7 | 1 / 1 | 7 / 3 | 1 / 9 | Only saccharide |
| Fructose | Lactic | N/A | 1% | 93% | 156% | 225% | 160% | 100% |
| | Tartaric | 34% | 46% | 128% | 106% | 148% | 60% | 100% |
| | Malic | 39% | 94% | 278% | 333% | 363% | 399% | 100% |
| | Citric | 32% | 0% | 134% | 291 % | 251% | 81% | 100% |
| | Succinic | 30% | 16% | 0% | 182% | 152% | 93% | 100% |

| Saccharide | Acid | Saccharide / Acid ratio by weight | | | | | | |
| | | Only acid | 1 / 9 | 3 / 7 | 1 / 1 | 7 / 3 | 1 / 9 | Only saccharide |
| Mannose | Lactic | N/A | 188% | 8947% | 2141% | 2888% | 363% | 100% |
| | Tartaric | 1388% | 1519% | 3543% | 2415% | 333% | 178% | 100% |
| | Malic | 1579% | 911% | 633% | 1185% | 1326% | 503% | 100% |
| | Citric | 1315% | 81% | 92% | 857% | 754% | 272% | 100% |
| | Succinic | 1204% | 2477% | 2658% | 2660% | 1395% | 226% | 100% |
| Ribose | Lactic | N/A | 99% | 5939% | 7655% | 9628% | 957% | 100% |
| | Tartaric | 1487% | 1039% | 4630% | 14304% | 11834% | 170% | 100% |
| | Malic | 1693% | 2022% | 7187% | 15682% | 17555% | 434% | 100% |
| | Citric | 1409% | 1748% | 3172% | 12115% | 5503% | 1663% | 100% |
| | Succinic | 1291% | 3378% | 9647% | 17868% | 14895% | 444% | 100% |

**Table 2:** *R. rhodochrous* "J1"

| Saccharide | Acid | Saccharide / Acid ratio by weight | | | | | | |
| | | Only acid | 1 / 9 | 3 / 7 | 1 / 1 | 7 / 3 | 1 / 9 | Only saccharide |
| Glucose | Lactic | 1963% | 2092% | 5069% | 4934% | 731% | 1836% | 100% |
| | Succinic | N/A | 1920% | 1270% | 4110% | 5548% | 1668% | 100% |

| Saccharide | Acid | Saccharide / Acid ratio by weight | | | | | | |
| | | Only acid | 1 / 9 | 3 / 7 | 1 / 1 | 7 / 3 | 1 / 9 | Only saccharide |
| Fructose | Lactic | 73% | 76% | 79% | 134% | 118% | 55% | 100% |
| | Succinic | N/A | 33% | 47% | 130% | 145% | 51% | 100% |

**Example 2**

**Measurement of nitrile hydratase activity**

Samples were taken from the cultivation medium of Example 1 and diluted 1:10 (v/v) with 50 mmol/l $KH_2PO_4$ buffer (pH 7,0). The solution obtained is further diluted 1:20 (v/v) with 50 mmol/l $KH_2PO_4$ buffer (pH 7,0) to achieve a final dilution factor of 1:200 for the cultivation medium.

Reaction:

875 µl of $KH_2PO_4$ buffer (pH 7,0) were pipetted in a 2 ml Eppendorf tube. 100 µl of the diluted cultivation medium were added and the mixture was pre-incubated in a thermomixer at 25 °C for 5 min at 500 rpm. After pre-incubation, the reaction was started by addition of 25 μl acrylonitrile. The reaction was carried out at 25 °C and 1,000 rpm for 10 min.

Preparation of samples:

After 10 min of reaction time, the reaction was quenched by transferring 300 μl of the reaction solution to a 1.5 ml Eppendorf tube which contained 300 μl of 1.4% (m/v) hydrochloric acid. The mixture was vortexed briefly and centrifuged in a table top centrifuge for 1 min at 10,000 rpm to separate the cells. 100 μl of the clear supernatant were mixed with 900 μl of water. The mixture was then analyzed by HPLC.

HPLC analysis:

- Column: Aqua 5μ C18 125A, 250 x 4,60 mm (Phenomenex)
- Oven temperature: 45 °C
- Injection volume:1 μl
- Detection: UV 210 nm
- Flow: 1,0 ml / min
- Solvent A: 25 mmol/l $KH_2PO_4$ pH 2,5
- Solvent B: Acetonitrile
- Seperation: 10% B (isocratic)
- Retention: Acrylamide: -3.6 min,
- HPLC-value should be between 0,25 and 1,25 mmol/l

The concentration of acrylamide determined by HPLC should be between 0.25 and 1.25 mmol/l. If this is not achieved directly, the cell concentration used in the reaction needs to be adjusted accordingly.

Determination of nitrile hydratase activity:

- c : amount of substance [mM] product (HPLC-value)
- t: incubation time [min], in this test 10 min
- k : dilution factor - total dilution from start sample to HPLC sample

calculation of activity in kU/l

$$\text{Activity (in kU/l)} = \frac{c}{t} \bullet k$$

- One activity unit [U] is defined as 1 μmol generated acrylamide during one minute reaction time.

**Claims**

1. Method for cultivating a nitrile hydratase producing microorganism, comprising the steps of:

    (a) contacting said microorganism with an aqueous composition comprising a saccharide (i) and an organic acid (ii); and
    (b) cultivating said microorganism in said aqueous composition of (a),

    wherein the ratio of saccharide (i) to organic acid (ii) is 3:7 to 7:3 by weight,
    wherein the saccharide is selected from the group consisting of glucose, fructose, mannose and ribose,
    wherein the organic acid is selected from the lactic acid, tartaric acid, malic acid, citric acid and succinic acid.

2. Composition for cultivating a nitrile hydratase producing microorganism, said composition comprising a saccharide (i) and an organic acid (ii)
    wherein the ratio of saccharide (i) to organic acid (ii) is 3:7 to 7:3 by weight,

wherein the saccharide is selected from the group consisting of glucose, fructose, mannose and ribose,
wherein the organic acid is selected from the lactic acid, tartaric acid, malic acid, citric acid and succinic acid.

3. Use of a composition comprising a saccharide (i) and an organic acid (ii) for cultivating a nitrile hydratase producing microorganism
wherein the ratio of saccharide (i) to organic acid (ii) is 3:7 to 7:3 by weight, wherein the saccharide is selected from the group consisting of glucose, fructose, mannose and ribose,
wherein the organic acid is selected from the lactic acid, tartaric acid, malic acid, citric acid and succinic acid.

4. Method of claim 1, composition of claim 2, or use of claim 3, wherein said saccharide (i) is a monosaccharide.

5. Method of claim 1 or 4, composition of claim 2 or 4, or use of claim 3 or 4, wherein the number of carbon atoms contained in said saccharide (i) is at least 5, preferably at least 6.

6. Method of any one of claims 1 or 4 to 5, composition of any one of claims 2 or 4 to 5, or use of any one of claims 3 to 5, wherein said organic acid (ii) comprises not more than 3 carboxyl groups, preferably not more than 2 carboxyl groups, most preferably not more than 1 carboxyl group.

7. Method of any one of claims 1 or 4 to 6, composition of any one of claims 2 or 4 to 6, or use of any one of claims 3 to 6, wherein said organic acid (ii) comprises not more than 6 carbon atoms, preferably not more than 4 carbon atoms, most preferably not more than 3 carbon atoms.

8. Method of any one of claims 1 or 4 to 7, composition of any one of claims 2 or 4 to 7, or use of any one of claims 4 to 7, wherein said microorganism belongs to the species Rhodococcus rhodochrous or Rhodococcus pyridinovorans.

9. Method of any one of claims 1 or 4 to 8, composition of any one of claims 2 or 4 to 8, or use of any one of claims 4 to 8, wherein said nitrile hydratase is encoded by a polynucleotide comprising a nucleotide sequence which is at least 70% identical to the nucleotide sequence shown in SEQ ID NO: 1 and/or SEQ ID NO: 3.

10. Method of any one of claims 1 or 4 to 9, composition of any one of claims 2 or 4 to 9, or use of any one of claims 4 to 9, wherein said nitrile hydratase has an amino acid sequence which is at least 70% identical to the amino acid sequence shown in SEQ ID NO: 2 and/or SEQ ID NO. 4.

11. Method of any one of claims 1 or 4 to 10, wherein said microorganism is dried after cultivation.


**Patentansprüche**

1. Verfahren zum Kultivieren eines Nitrilhydratase produzierenden Mikroorganismus, umfassend die Schritte:

   (a) Inkontaktbringen des Mikroorganismus mit einer wässrigen Zusammensetzung umfassend ein Saccharid (i) und eine organische Säure (ii); und
   (b) Kultivieren des Mikroorganismus in der wässrigen Zusammensetzung von (a),

   wobei das Verhältnis von Saccharid (i) zu organischer Säure (ii) 3:7 bis 7:3 nach Gewicht beträgt,
   wobei das Saccharid aus der Gruppe bestehend aus Glucose, Fructose, Mannose und Ribose ausgewählt ist,
   wobei die organische Säure aus Milchsäure, Weinsäure, Äpfelsäure, Zitronensäure und Bernsteinsäure ausgewählt ist.

2. Zusammensetzung zum Kultivieren eines Nitrilhydratase produzierenden Mikroorganismus, wobei die Zusammensetzung ein Saccharid (i) und eine organische Säure (ii) umfasst,
   wobei das Verhältnis von Saccharid (i) zu organischer Säure (ii) 3:7 bis 7:3 nach Gewicht beträgt,
   wobei das Saccharid aus der Gruppe bestehend aus Glucose, Fructose, Mannose und Ribose ausgewählt ist,
   wobei die organische Säure aus Milchsäure, Weinsäure, Äpfelsäure, Zitronensäure und Bernsteinsäure ausgewählt ist.

3. Verwendung einer Zusammensetzung umfassend ein Saccharid (i) und eine organische Säure (ii) zum Kultivieren eines Nitrilhydratase produzierenden Mikroorganismus,

wobei das Verhältnis von Saccharid (i) zu organischer Säure (ii) 3:7 bis 7:3 nach Gewicht beträgt, wobei das Saccharid aus der Gruppe bestehend aus Glucose, Fructose, Mannose und Ribose ausgewählt ist, wobei die organische Säure aus Milchsäure, Weinsäure, Äpfelsäure, Zitronensäure und Bernsteinsäure ausgewählt ist.

4. Verfahren nach Anspruch 1, Zusammensetzung nach Anspruch 2 oder Verwendung nach Anspruch 3, wobei es sich bei dem Saccharid (i) um ein Monosaccharid handelt.

5. Verfahren nach Anspruch 1 oder 4, Zusammensetzung nach Anspruch 2 oder 4 oder Verwendung nach Anspruch 3 oder 4, wobei die Anzahl an Kohlenstoffatomen in dem Saccharid (i) mindestens 5, vorzugsweise mindestens 6, beträgt.

6. Verfahren nach einem der Ansprüche 1 oder 4 bis 5, Zusammensetzung nach einem der Ansprüche 2 oder 4 bis 5 oder Verwendung nach einem der Ansprüche 3 bis 5, wobei die organische Säure (ii) nicht mehr als 3 Carboxylgruppen, vorzugsweise nicht mehr als 2 Carboxylgruppen, am meisten bevorzugt nicht mehr als 1 Carboxylgruppe, umfasst.

7. Verfahren nach einem der Ansprüche 1 oder 4 bis 6, Zusammensetzung nach einem der Ansprüche 2 oder 4 bis 6 oder Verwendung nach einem der Ansprüche 3 bis 6, wobei die organische Säure (ii) nicht mehr als 6 Kohlenstoffatome, vorzugsweise nicht mehr als 4 Kohlenstoffatome, am meisten bevorzugt nicht mehr als 3 Kohlenstoffatome, umfasst.

8. Verfahren nach einem der Ansprüche 1 oder 4 bis 7, Zusammensetzung nach einem der Ansprüche 2 oder 4 bis 7 oder Verwendung nach einem der Ansprüche 4 bis 7, wobei der Mikroorganismus zu den Spezies Rhodococcus rhodochrous oder Rhodococcus pyridinovorans gehört.

9. Verfahren nach einem der Ansprüche 1 oder 4 bis 8, Zusammensetzung nach einem der Ansprüche 2 oder 4 bis 8 oder Verwendung nach einem der Ansprüche 4 bis 8, wobei die Nitrilhydratase durch ein Polynukleotid codiert wird, das eine Nukleotidsequenz umfasst, die zu mindestens 70 % mit der in SEQ ID NR: 1 und/oder SEQ ID NR: 3 gezeigten Nukleotidsequenz identisch ist.

10. Verfahren nach einem der Ansprüche 1 oder 4 bis 9, Zusammensetzung nach einem der Ansprüche 2 oder 4 bis 9 oder Verwendung nach einem der Ansprüche 4 bis 9, wobei die Nitrilhydratase eine Aminosäuresequenz aufweist, die zu mindestens 70 % mit der in SEQ ID NR: 2 und/oder SEQ ID NR: 4 gezeigten Aminosäuresequenz identisch ist.

11. Verfahren nach einem der Ansprüche 1 oder 4 bis 10, wobei der Mikroorganismus nach der Kultivierung getrocknet wird.

**Revendications**

1. Procédé de culture d'un microorganisme produisant de la nitrile hydratase, comprenant les étapes suivantes :

   (a) mise en contact dudit microorganisme avec une composition aqueuse comprenant un saccharide (i) et un acide organique (ii) ; et
   (b) culture dudit microorganisme dans ladite composition aqueuse de (a),

   dans lequel le rapport du saccharide (i) sur l'acide organique (ii) est de 3/7 à 7/3 en poids,
   dans lequel le saccharide est sélectionné dans le groupe consistant en le glucose, le fructose, le mannose et le ribose,
   dans lequel l'acide organique est sélectionné parmi l'acide lactique, l'acide tartrique, l'acide malique, l'acide citrique et l'acide succinique.

2. Composition pour la culture d'un microorganisme produisant de la nitrile hydratase, ladite composition comprenant un saccharide (i) et un acide organique (ii) ,
   dans laquelle le rapport du saccharide (i) sur l'acide organique (ii) est de 3/7 à 7/3 en poids,
   dans laquelle le saccharide est sélectionné dans le groupe consistant en le glucose, le fructose, le mannose et le ribose,
   dans laquelle l'acide est sélectionné parmi l'acide lactique, l'acide tartrique, l'acide malique, l'acide citrique et l'acide succinique.

3. Utilisation d'une composition comprenant un saccharide (i) et un acide organique (ii) pour la culture d'un microorganisme produisant une nitrile hydratase
dans laquelle le rapport du saccharide (i) sur l'acide organique (ii) est de 3/7 à 7/3 en poids,
dans laquelle le saccharide est sélectionné dans le groupe consistant en le glucose, le fructose, le mannose et le ribose,
dans laquelle l'acide est sélectionné dans le groupe consistant en l'acide lactique, l'acide tartrique, l'acide malique, l'acide citrique et l'acide succinique.

4. Procédé selon la revendication 1, composition selon la revendication 2, ou utilisation selon la revendication 3, dans lequel ledit saccharide (i) est un monosaccharide.

5. Procédé selon la revendication 1 ou 4, composition selon la revendication 2 ou 4, ou utilisation selon la revendication 3 ou 4, dans lequel le nombre d'atomes de carbone contenus dans ledit saccharide (i) est au moins 5, de préférence au moins 6.

6. Procédé selon l'une quelconque des revendications 1 ou 4 à 5, composition selon l'une quelconque des revendications 2 ou 4 à 5, ou utilisation selon l'une quelconque des revendications 3 à 5, dans lequel ledit acide organique (ii) ne comprend pas plus de 3 groupes carboxyle, de préférence pas plus de 2 groupes carboxyle, de manière préférée entre toutes pas plus de 1 groupe carboxyle.

7. Procédé selon l'une quelconque des revendications 1 ou 4 à 6, composition selon l'une quelconque des revendications 2 ou 4 à 6, ou utilisation selon l'une quelconque des revendications 3 à 6, dans lequel ledit acide organique (ii) ne comprend pas plus de 6 atomes de carbone, de préférence pas plus de 4 atomes de carbone, de manière préférée entre toutes pas plus de 3 atomes de carbone.

8. Procédé selon l'une quelconque des revendications 1 ou 4 à 7, composition selon l'une quelconque des revendications 2 ou 4 à 7, ou utilisation selon l'une quelconque des revendications 4 à 7, dans lequel ledit microorganisme appartient à l'espèce *Rhodococcus rhodochrous* ou *Rhodococcus pyridinovorans.*

9. Procédé selon l'une quelconque des revendications 1 ou 4 à 8, composition selon l'une quelconque des revendications 2 ou 4 à 8, ou utilisation selon l'une quelconque des revendications 4 à 8, dans lequel ladite nitrile hydratase est codée par un polynucléotide comprenant une séquence nucléotidique qui présente une identité d'au moins 70 % avec la séquence nucléotidique présentée dans SEQ ID NO : 1 et/ou SEQ ID NO : 3.

10. Procédé selon l'une quelconque des revendications 1 ou 4 à 9, composition selon l'une quelconque des revendications 2 ou 4 à 9, ou utilisation selon l'une quelconque des revendications 4 à 9, dans lequel ladite nitrile hydratase a une séquence d'acides aminés qui présente une identité d'au moins 70 % avec la séquence d'acides aminés présentée dans SEQ ID NO : 2 et/ou SEQ ID NO : 4.

11. Procédé selon l'une quelconque des revendications 1 ou 4 à 10, dans lequel ledit microorganisme est séché après la culture.

## REFERENCES CITED IN THE DESCRIPTION

**Patent documents cited in the description**

- US 4048226 A
- US 3597481 A
- FR 2835531
- EP 1283256 B1

- US 5525711 A
- US 4711955 A
- US 5792608 A
- EP 302175 A

**Non-patent literature cited in the description**

- *CHEMICAL ABSTRACTS,* 2391-37-5
- **PRASAD.** *Biotechnology Advances,* 2010, vol. 28 (6), 725-741
- **KOBAYASHI.** *Nature Biotechnology,* 1998, vol. 16, 733-736
- **LEONOVA.** *Applied Biochem Biotechnol,* 2000, vol. 88, 231-241

- **PEI.** *Biotechnology letters,* 2013, vol. 35, 1419-1424
- **SAMBROOK ; RUSSELL.** Molecular Cloning: A Laboratory Manual. CSH Press, 2001
- **GAMPER.** *Nucleic Acids Research,* 2000, vol. 28, 4332-4339